# EUROPEAN PATENT SPECIFICATION

(11) **EP 0 463 073 B1**
(45) Date of publication and mention of the grant of the patent: **09.12.1998**
(21) Application number: 90905191.4
(22) Date of filing: 16.03.1990
(51) Int. Cl.: C12P 21/02, C12N 9/50, C12N 15/57, A61K 39/002, A61K 39/095

(54) **IMMUNOGLOBULIN A1 PROTEASES (IgA1 PROTEASES), METHOD OF GENE-TECHNOLOGICALLY PRODUCING THIS TYPE OF ENZYME AND VACCINES CONTAINING THE ENZYMES AND FRAGMENTS THEREOF FOR IMMUNIZING AGAINST BACTERIAL MENINGITIS AND OTHER DISEASES CAUSED BY IgA1 PROTEASE-PRODUCING BACTERIA**
IMMUNGLOBULIN-A1-PROTEASEN (IGA1-PROTEASEN), VERFAHREN ZUR GENTECHNOLOGISCHEN HERSTELLUNG DIESES ENZYMTYPS SOWIE IMPFSTOFFE, DIE DIE ENZYME UND FRAGMENTE DAVON ENTHALTEN, ZUR IMMUNISIERUNG GEGEN BAKTERIELLE MENINGITIS UND ANDERE, DURCH IGA1-PROTEASEPRODUZIERENDE BAKTERIEN VERURSACHTE KRANKHEITEN
PROTEASES D'IMMUNOGLOBULINE A1 (PROTEASES IgA1), PROCEDE DE PRODUCTION PAR GENIE GENETIQUE DE CE TYPE D'ENZYME ET VACCINS CONTENANT LES ENZYMES AINSI QUE DES FRAGMENTS DE CELLES-CI POUR L'IMMUNISATION CONTRE LA MENINGITE BACTERIENNE AINSI QUE D'AUTRES MALADIES PROVOQUEES PAR DES BACTERIES PRODUISANT LA PROTEASE IgA1

(30) Priority: 17.03.1989 DK 130889
(43) Date of publication of application: 02.01.1992
(73) Proprietor: KILIAN, Mogens, DK-8240 Risskov (DK); POULSEN, Knud, DK-9260 Viby J. (DK)
(72) Inventor: KILIAN, Mogens, DK-8240 Risskov (DK); POULSEN, Knud, DK-9260 Viby J. (DK)
(74) Representative: Christiansen, Ejvind
(86) International application number: DK9000073
(87) International publication number: WO9011367

(56) References cited:
- CHEMICAL ABSTRACTS, Volume 107, No. 21, 23 November 1987, (Columbus, Ohio, US), GRUNDY, FRANK J. et al: "Haemophilus influenzae immunoglobulin A1 protease genes: cloning by plasmid integration-excosion, comparative analyses, and localization of secretion determinants", see page 207, Abstract 192238f, & J. Bacteriol. 1987, 169(10), 4442-44509.
- CHEMICAL ABSTRACTS, Volume 104, No. 15, 14 April 1986, (Columbus, Ohio, US), FISHMAN, Y et al: "Cloning of the type 1 immunoglobulin A1 protease from Neisseria gonorrhoeae and secretion of the enzyme from Escherichia coli", see page 184, Abstract 124204e, & Pathog.Neisseriae, Proc.Int.Symp., 4th 1984, 164(8), 9.
- CHEMICAL ABSTRACTS, Volume 99, No. 5, 1 August 1983, (Columbus, Ohio, US), BRICKER, J. et al: "IgA1 proteases of Haemophilus influenzae: cloning and characterization in Escherichia coli K-12", see page 132, Abstract 33850z, & Proc.Natl.Acad.Sci.U.S.A. 1983, 80( 9), 2681-26859.
- CHEMICAL ABSTRACTS, Volume 98, No. 91, 28 February 1983, (Columbus, Ohio, US), KOOMEY, J. MICHAEL et al: "Genetic and biochemical analysis of gonococcal IgA1 protease: cloning in Escherichia coli and construction of mutants of gonococci that fail to produce the activity", see page 145, Abstract 66414t, & Proc.Natl. Acad.Sci.U.S.A. 1982, 79(24).
- Infection and Immunity 56(8), 2060-2068 (1988)
- Journal of Bacteriology 169(6), 2781-2792 (1987)

## Description

The present invention relates to immunoglobulin A1 proteases (IgA1 proteases) and immunogenic fragments thereof with the ability to induce neutralizing antibodies against IgA1 proteases produced by pathogenic Haemophilus influenzae serotype b, Neisseria meningitidis and/or Neisseria Gonorrhea. The invention also relates to a method of producing such IgA1 proteases. Further, the invention relates to vaccines containing the inventive IgA1 proteases, in particular for the prevention of meningitis, but also for immunizing against allergic diseases, gonorrhoea and other diseases caused by IgA protease-producing bacteria.

### Background of the invention:

The immunoglobulin A1 proteases (IgA1 proteases) are extracellular bacterial enzymes which specifically cleave the heavy chain of human IgA1 molecules in the hinge region. Production of IgA1 protease seems to be correlated with the ability of the bacteria to infect and in some instances invade mucosal membranes. Notably, the three leading causes of bacterial meningitis (Haemophilus influenzae serotype b, Neisseria meningitidis, and Streptococcus pneumoniae) secrete IgA1 proteases whereas closely related non-pathogenic species are devoid of similar enzyme activity. IgA1 protease production is also a property of certain bacterial species causing vaginal and urinary tract infections, such as Neisseria gonorrhoeae and Ureaplasma urealyticum. In addition, several oral bacteria involved in dental plaque formation (Streptococcus sanguis, Streptococcus oralis, and Streptococcus mitis biovar 1 (the two latter species are former parts of Streptococcus mitior) and in the pathogenesis of periodontal diseases (Bacteroides and Capnocytophaga species) secrete IgA1 proteases (Kilian, M. and Reinholdt, J.: "Interference with IgA defence mechanisms by extracellular enzymes", p. 173-208 in C.S.F. Easmon and J. Jeljaszewics (ed.), 1986, Medical Microbiology vol. 5, Academic Press, Inc., London; see also Mulks, M. H.: "Microbial IgA proteases" p. 81-104 in I. A. Holder (ed.), 1985, Bacterial Enzymes and Virulence, CRC Press, and Plaut, A.G., Ann. Rev. Microbiol. 37, 603-622 (1983)). IgA1 proteases are believed to be important virulence factors as they allow bacteria to interfere with the protective mechanisms of IgA1, which is the predominant immunoglobulin class on relevant mucosal surfaces (Kett, K. et al., J. Immunol. 136, 3631-3635 (1986)).

Because of the specificity of IgA1 proteases for human IgA1 experiments with animals cannot confirm the pathogenic significance of these enzymes. However, extensive cleavage of IgA in secretions and other body fluids from patients infected with said bacteria is observed. Furthermore, it has been shown that nasopharyngeal secretions from children with a history of allergic diseases, more than from healthy children, show extensive IgA1 protease-induced degradation of the IgA, which was to form a protective barrier to potential allergens and microorganisms (Christensen, C.H. and Kilian, M., Acta Path. Microbiol. Immunol. Scand. 92C, 85-87, 1984).

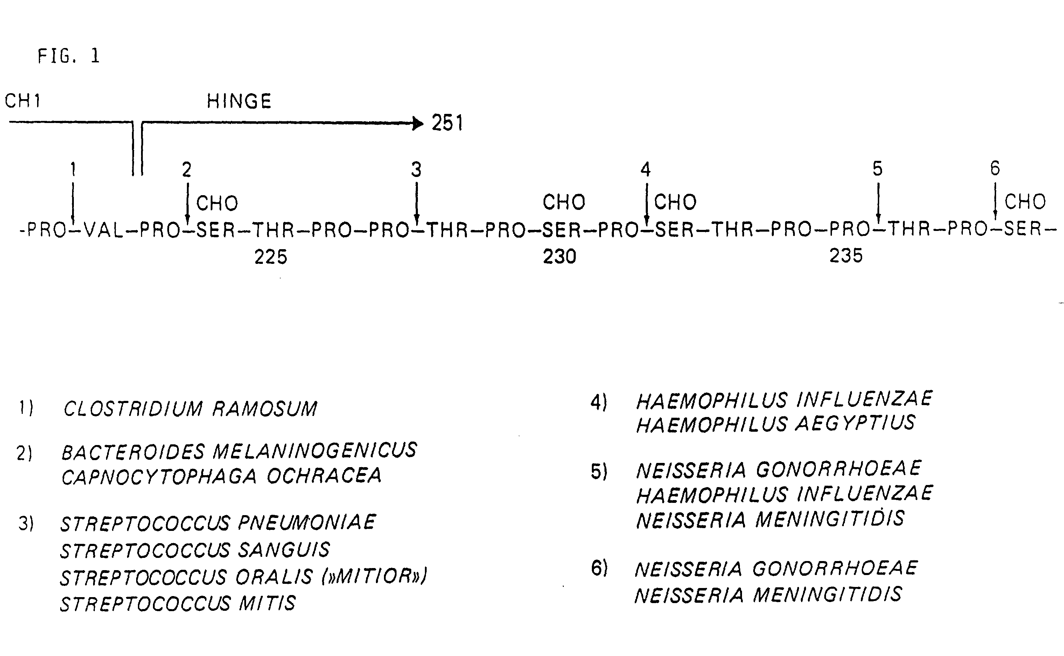

Several enzymatically different types of IgA1 proteases have been reported, each cleaving the human IgA1 molecule at a specific site in the hinge region, as shown in fig. 1 (Kilian, M. and Reinholdt, J., supra). In Haemophilus influenzae at least two such distinct enzymes are known (type 1 cleaving the PRO-SER peptide bond at position 231-232 and type 2 cleaving the PRO-SER peptide bond at position 235-236). Likewise, Neisseria meningitidis and Neisseria gonorrhoeae may secrete a type 1 or a type 2 protease (see fig. 1).

An IgA1 protease produced by cloning the gene for the IgA1 protease from a Haemophilus influenzae strain in Eschrichia coli is disclosed in Bricker et al., Proc. Natl. Acad. Sci. USA 80, 2681-2685 (1983). However, the IgA1 protease in question originates from a Haemophilus influenzae strain (serotype d), which is unrelated to infectious diseases. It is true that the citation describes the successful expression of the IgA1 protease from this strain in E. coli, but the cloned gene was not sequenced and the resulting IgA1 protease cannot be used as a vaccine against bacterial meningitis and other infectious diseases caused by H. influenzae serotype b strains.

Grundy et al., J. Bacteriology 169, 4442-4450 (1987) disclose cloning of the IgA1 protease gene from Haemophilus influenzae serotype c and d in E. coli. The resulting IgA1 proteases are unfit to be used in vaccines against infectious diseases caused by H. influenzae serotype b strains for the same reasons as outlined above.

### Polymorphism of IgA1 proteases:

IgA1 proteases from different bacteria have been compared by genetical and serological methods. Hybridization experiments using the bulk of a gonococcal IgA1 protease gene as probe revealed substantial homology (>78%) between IgA1 protease genes from different gonococcal and meningococcal strains. More surprisingly, an estimated 67-75% homology between the gonococcal gene and that from a Haemophilus influenzae strain (Rd) was observed (Koomey, J.M. and Falkow, S., Infect. Immun. 43, 101-107, 1984). However, the use of restriction enzyme mapping of IgA1 protease genes revealed a considerable degree of polymorphism even between strains belonging to the same species (Halter, R. et al. EMBO J. 3, 1595-1601, 1984; Bricker, J. et al., Infect. Immun. 4, 370-374, 1985; Mulks, M.H. and Knapp, J.S., Infect. Immun. 55, 931-931, 1987). Within a large collection of Haemophilus influenzae serotype b strains four different IgA1 protease gene types were detected with restriction enzyme analyses (Poulsen, K., Hjorth, J.P. and Kilian, M. Infect. Immun. 56, 987-992, 1988).

The extensive polymorphism has been confirmed by comparing IgA1 proteases with neutralizing antibodies raised in rabbits. When immunized with IgA1 protease preparations rabbits respond with antibodies capable of neutralizing the enzyme used for immunization. With such antibodies at least 15 antigenically different types of IgA1 proteases have been identified among H.influenzae isolates (Kilian, M. et al., Molec. Immunol. 20, 1051-1058, 1983) and two types among H.influenzae serotype b (Poulsen K. et al. supra). A partial antigenic relationship has been observed between one out of two strains of N.meningitidis and one strain of N.gonorrhoeae (Kilian, M., et al., Ann. N.Y. Acad. Sci. 409, 612-624, 1983). Furthermore, type 1 and type 2 IgA1 proteases of Neisseriae can be distinguished on the basis of their relative inactivation by sera from patients recovering from meningococcal infection by either type 1 or type 2 protease producing bacteria (Stafford and Plaue, Abstr. Annu. Met. Am. Soc. Microbiol. 1982, B125, p. 38).

### Cloning of IgA1 protease genes

The genes encoding IgA1 protease (iga genes) produced by Neisseria gonorrhoeae type 1 (Fishman, Y., Bricker, J., Gilbert, J.V., Plaut, A.G. and Wright, A.: "Cloning of the type 1 immunoglobulin A1 protease from Neisseria gonorrhoeae and secretion of the enzyme from Escherichia coli", 1985, p. 164-168 in G. K. Schoolnik (ed.), The pathogenic Neisseria, Am. Soc. Microbiology, Washington DC) and type 2 (Koomey, J. M., Gill, R.E. and Falkow, S., Proc. Natl. Acad. Sci. USA, 79, 7881-7885, 1982; Halter, R., Pohlner, J. and Meyer, T.F., EMBO, J. 3, 1595-1601, 1984), Haemophilus influenzae type 1 of serotype d origin (Bricker, J. et. al., supra; and Koomey, J. M. and Falkow, S., supra) and type 2 of serotype c origin (Grundy, J. F. et. al., supra), Neisseria meningitidis (Koomey and Falkow, supra and Streptococcus sanguis (Gilbert, J.V. et al., Infect. Immun. 56, 1961-1966 (1988)) have been cloned.

E. coli transformed with each of these iga genes express IgA1 protease activity and the E. coli hosts, which harbour the H. influenzae and the N. gonorrhoeae iga genes, have been found to excrete the protease. At present, an example of the type 2 IgA1 protease gene from N. gonorrhoeae is the only iga gene for which the nucleotide sequence is known (Pohlner, J. et al., Nature 325, 458-462 (1987); see also DE OS 36 22 221). The deduced primary structure of the protein together with analyses of intermediate IgA1 protease precursors have revealed that the protease is expressed as a preprotein containing a signal peptide, the structural protease and a helper protein, which is autoproteolytically processed during the secretion process. Despite the similarity between the enzymes from Neisseria sp. and H.influenzae it has been concluded that the Haemophilus enzyme is not transported by the mechanism used for secretion of the Neisseria IgA1 protease (Fishman et. al., supra; see also DE OS 36 22 221).

A first aspect of the invention is based on the surprising finding that the gene coding for an IgA1 protease from Haemophilus influenzae serotype b can be cloned in E. coli to achieve extracellular enzyme secretion, thus making it possible to extract the enzyme from the cultivation medium.

Previously, fragments of the cloned type 1 IgA1 protease gene of H. influenzae serotype d origin have been used as probes in Southern blot experiments to study the restriction site polymorphism of the iga gene among different H. influenzae serotype b strains (Poulsen, K., Hjort, J.P. and Kilian, M., Infect. Immun. 56 987-992 (1988)). Based upon these iga gene restriction patterns the strains could be divided into four groups, which correlated with previously observed clusters of multilocus genotypes (electrophoretic types). Three of the iga gene restriction types, which appeared to represent 98% of the H. influenzae serotype b population, exclusively contained strains of the same unique IgA1 protease "inhibition type" and therefore could form the basis for the development of a vaccine against H. influenzae meningitis.

Experiments with inhibitory antibodies have shown that the three gene types direct the production of proteases with a mutual epitope, which is found in each of the three proteases.

A second aspect of the invention is based on the finding that an IgA1 protease from N. meningitidis has the ability to induce neutralizing antibodies against IgA1 proteases of a number of known genotypes of pathogenic N. meningitidis and therefore is applicable in vaccines against meningococcal and gonococcal meningitis.

### Detailed Description of Aspect 1 (H. influenzae)

In the present invention H. influenzae strain HK368 was chosen as representative for the most common serotype b iga gene type (Poulsen, K. et al., supra). In the following the cloning and sequencing of the iga gene from this strain is described. Comparing the deduced amino acid sequence of this protease to that of N. gonorrhoeae reveals interesting regions of homology.

In the following the invention is illustrated in more detail, reference being made to the drawing in which
- fig. 1: shows the primary structure of the hinge region of human IgA1;
- fig. 2: shows the structure of the H. influenzae serotype b strain HK368 IgA1 protease gene;
- fig. 3: shows the nucleotide sequence of the cloned iga gene from H. influenzae serotype b strain HK368;
- fig. 4: illustrates the amino acid homologies between the deduced H. influenzae iga gene product and the N. gonorrhoeae iga gene product, and
- Fig. 5: shows a physical map of lambda HF13iga-1 expressing the meningococcal IgA1 protease gene from a N. meningitidis strain of serogroup Y, subtype 2c (designated HF13).

The invention is illustrated in more detail in the following. When carrying out the experiments the following materials and methods were used:

Bacterial strains. The wild-type H. influenzae serotype b strain HK368 isolated from cerebrospinal fluid from a meningitis patient was the source of whole-cell DNA for molcular cloning. It was grown in Brain Heart Infusion broth (Difco, Detroit, Mich.) supplemented with hemin and nicotinamide adenine dinucleotide (NAD) (Kilian, M. J. Gen. Microbiol. 93, 9-62 (1976)).

E. coli strain K802 was used for propagation of lambda phages. The M13mp19 phage and recombinant derivatives hereof were propagated in E. coli JM109 as described by Yanisch-Perron, C. et al., Gene 33, 103-119 (1985).

Enzymes and chemicals. Restriction endonucleases were purchased from Amersham International (Amersham, England) and Boehringer GmbH (Mannheim, FRG). T4 ligase, Proteinase K, RNase A, and DNase I were obtained from Boehringer; DNA polymerase I, DNA polymerase I Klenow fragment, and radioactively labelled deoxynucleotides were from Amersham. Exonuclease III came from Pharmacia (Uppsala, Sweden), lysozyme from Sigma (St. Louis, MS), M13 pentadecamer primer from New England Biolabs (Beverly, CA), and Sequenase DNA sequencing kit was from United States Biochemical Corporation (Cleveland, OH).

Cloning of H. influenzae HK 368 DNA in phage λL47.1. DNA from H. influenzae isolated as described previously (Poulsen, K. et al., supra) was partially digested with Sau3A and fractionated by agarose gel electrophoresis. Fragments ranging in size from 12 to 20 kilobases (kb) were extracted from the gel by electroelution and cloned using λL47.1 as BamHI substitution vector.

Phages packaged in vitro as described by Maniatis et al., "Molecular Cloning. A laboratory Manual", Cold Spring Harbor Laboratory, N.Y., were plated on E. coli strain K802 and positive plaques identified by in situ hybridization. Positive plaques were purified by replating on E. coli strain K802 and DNA isolated from 20 ml phage cultures (Mikkelsen, B.M. et al., Biochem. Genet. 23, 511-524 (1985)).

Nucleic acid hybridizations and DNA manipulations. As label in the hybridizations (α-³²P)-dATP, which was incorporated into the DNA probes by nick-translation, was used (Rigby, P.W.J. et al., J. Mol. Biol. 11, 237-251 (1977)). The in situ probing of phages fixed on nitrocellulose filters was carried out as described by Benton and Davies, Science 196, 180-182 (1977) and Southern blot experiments were performed as described previously (Poulsen, K. et al., supra). Fragments of plasmid pVD116 containing H. influenzae type d iga gene were used as probes. Subcloning into M13mp19 of restriction fragments isolated by electroelution and DNA purifications and manipulations were carried out as described by Maniatis et al., supra.

Progressive deletions of recombinant M13mp19 phages for the sequencing procedure were produced by varying the time length of Exonuclease III digestion of BamHI/SacI opened replicative form DNA (Yanisch-Perron et al., supra). For removal of the resulting single stranded ends S1 nuclease was used instead of Exonuclease III.

DNA sequencing. The sequences of individual clones were determined by the dideoxynucleotide chain termination method (Sanger, F. et al., Proc. Natl. Acad. Sci. USA 74, 5463-5467 (1977) and Biggin, M.D. et al., ibid. 80, 3963-3965 (1983)) using the 15-mer universal primer and (α-³⁵S)-dATP. For some of the sequences Sequenase DNA sequencing kit was used according to the manufacturer's recomendations. The program of Larson and Messing (Nucl. Acids Res. 10, 39-49 (1982)) was used to assemble to sequence data using an Apple IIe computer.

Sequence alignment. Computer assisted analysis of amino acid sequence data was performed by the program described in J. Mol. Biol. 195, 43-61 (1987).

Assay for IgA1 protease activity. IgA1 protease activity in phage lysates was detected by mixing 1 vol. of the lysate supernatant with 1 vol. of a 2 mg/ml solution of hyman myeloma IgA1. After overnight incubation, cleavage of the substrate IgA1 was demonstrated by immunoelectrophoresis as described by Kilian, M. et al., Mol. Immunol. 220, 1051-1058 (1983)).

Immunization and inhibition assay. The supernatant of a E. coli lysate of a recombinant phage containing the iga gene was used for immunization of a rabbit. The immunization procedure and protease inhibition assay was as described previously (Kilian, M. et al., supra).

The following results were obtained:

### Cloning of the H. influenzae serotype b iga gene in E. coli.

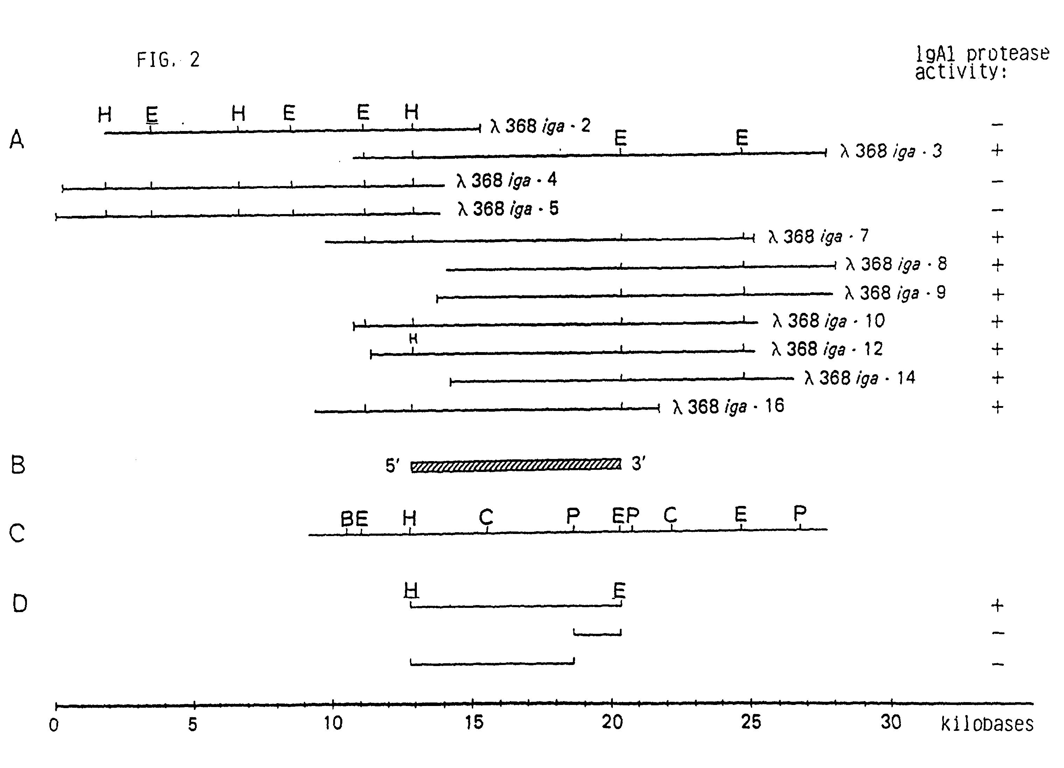

The E. coli plasmid pVD116 contains the iga gene from H. influenzae strain Rd /be (Koomey, J. M., Proc. Natl. Acad. Sci. USA 79, 7881-7885 (1982)). A 2.0 kb ClaI/PstI fragment of this plasmid containing the 5' end of the iga gene of serotype d origin was used as radioactively labelled probe to isolate the iga gene from a lambda-phage library of H. influenzae serotype b strain HK368 DAN using the vector λL47.1. Replica filters of approximately 2x10⁻³ recombinant λ-phages from the non-amplified library were screened with the probe. Sixteen positive clones labelled λ368iga-1 to λ368iga-16 were found. Assuming that iga is a single copy gene, this frequency is in acceptable agreement with the estimated H. influenzae genome size of 1.8x10⁻³ kb and an average insert of 15 kb in the recombinant λ-phages. Eleven of these positive clones were purified and recombinant phage DNA isolated. The localization of recognition sites for the restriction endonucleases EcoRI and HindIII in the insert DNA were determined by complete and partial digestions of DNA from the individual λ368iga phages as previously described (Mikkelsen, B.M. et al., supra). In addition, DNA from λ368iga-8 and λ368iga-16 were mapped with respect to the restriction enzymes BamHI, ClaI, and PstI. The restriction maps of the inserts overlap as shown in Fig. 2. This strongly suggests that the iga gene in H. influenzae strain HK368 is a single copy gene.

Localization of the iga gene. The previously described 5'-iga specific probe together with a 3'-iga specific 2.8 kb PstI/EcoRI fragment of pVD116 were hybridized to replica Southern blots of EcoRI, HindIII, and Sau3A restricted DNA from each of the eleven λ368iga clones analysed. Thereby, the iga gene was deduced to be localized within a 7.5 kb EcoRI/HindIII fragment and oriented as shown in Fig. 2.

The restriction enzyme Sau3A sites at the insert vector junctions are preserved during the cloning. For seven of the eleven recombinant clones analysed the size and number of DNA fragments generated by Sau3A and hybridizing to the two iga probes were identical. The same pattern of bands was observed in Southern blots of Sau3A digested whole-cell DNA from H. influenzae strain HK368 hybridized with the same two probes (data not shown). This indicated that not rearrangements of the hybridizing region in these lambda phages had occurred during the cloning procedure. In addition, this result confirms that the same iga-specific sequence had been cloned in these phages.

Liquid lysates from these seven recombinant phage clones were found to possess IgA1 protease activity. This confirms the cloning of the iga gene. In the lysates from three of the eleven clones, i.e. λ368iga-2, λ368iga-4, and λ368iga-5, IgA1 cleaving activity could not be detected. DNA from these three clones lacked some of the Sau3A fragments found in strain HK368 genomic DNA, which hybridized to the 3'-iga probe, and therefore only contain the 5' part of the iga gene (see Fig. 2). The clone λ368iga-8, which encodes IgA1 protease activity, lacked some of the HK368 genomic Sau3A fragments hybridizing to the 5'-iga probe. This indicates that the plasmid pVD116, which was used as probe, contains sequences originating from the H. influenzae serotype d genome beyond the iga gene.

The 7.5 kb EcoRI/HindIII fragment of λ368iga-16, which contains the iga gene, was subcloned into M13mp19. In the supernatant of liquid cultures of E. coli JM109 transformed with this recombinant M13 phage we detected IgA1 protease activity at a level comparable to the IgA1 cleaving activity found in the supernatant of H. influenzae strain HK368 cultures. Because the iga gene was cloned in the opposite direction of the lac Z gene in M13mp19 this result confirms that the H. influenzae iga gene is expressed in E. coli. It also indicates that the secretion mechanisms of the IgA1 protease function in E. coli since the M13 phages do not lyse the bacterial cells.

Crude extract from a λ368iga-16 lysate was used to immunize rabbits. The immunoglobulin fraction of the resulting antisera had complete inhibitory effect on the IgA1 protease activity from H. influenzae strain HK368 culture supernatant. This shows that H. influenzae strain HK368 does not secrete any IgA1 protease unrelated to the one encoded by the iga gene cloned in λ368iga-16.

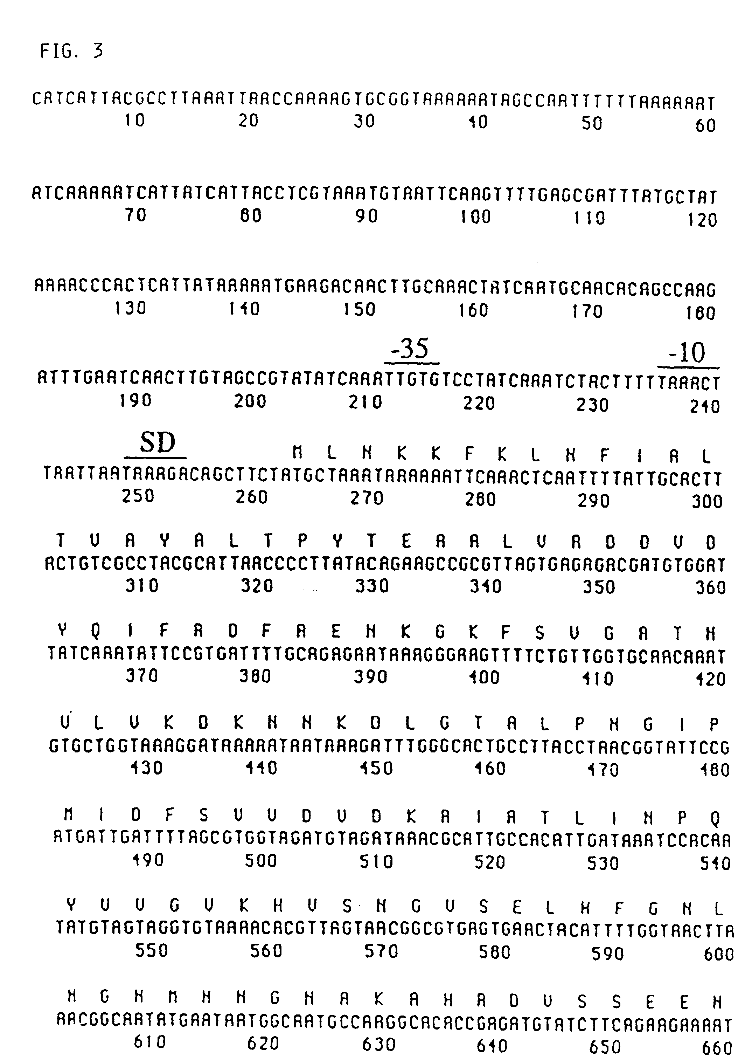

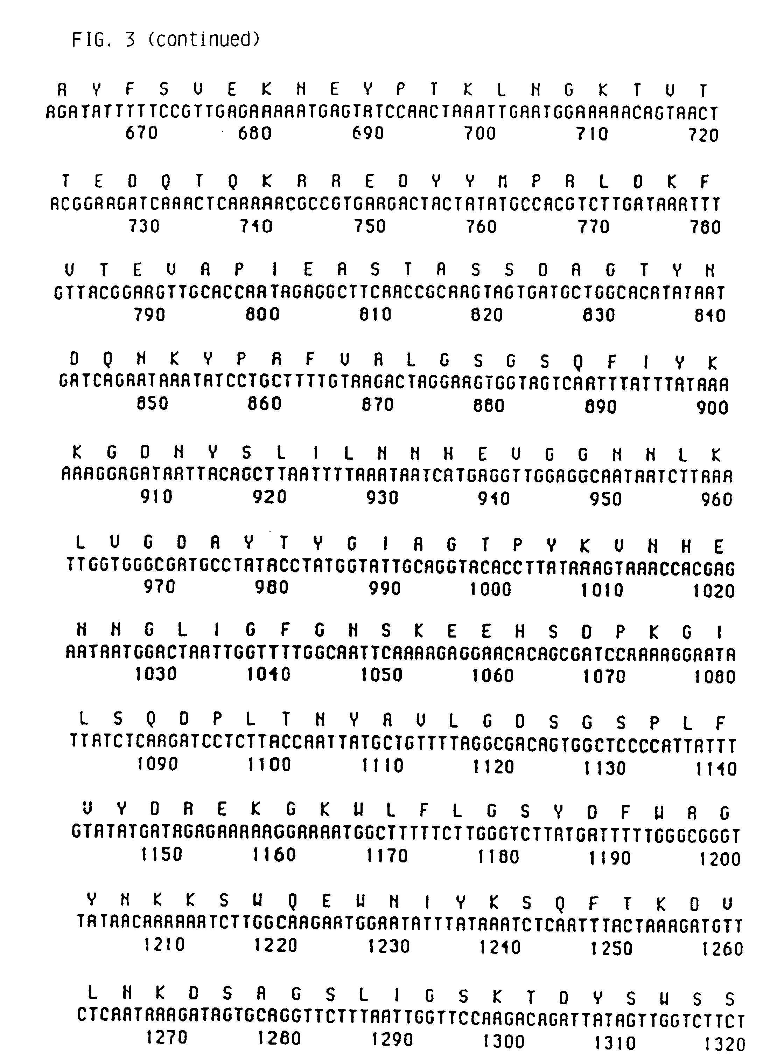

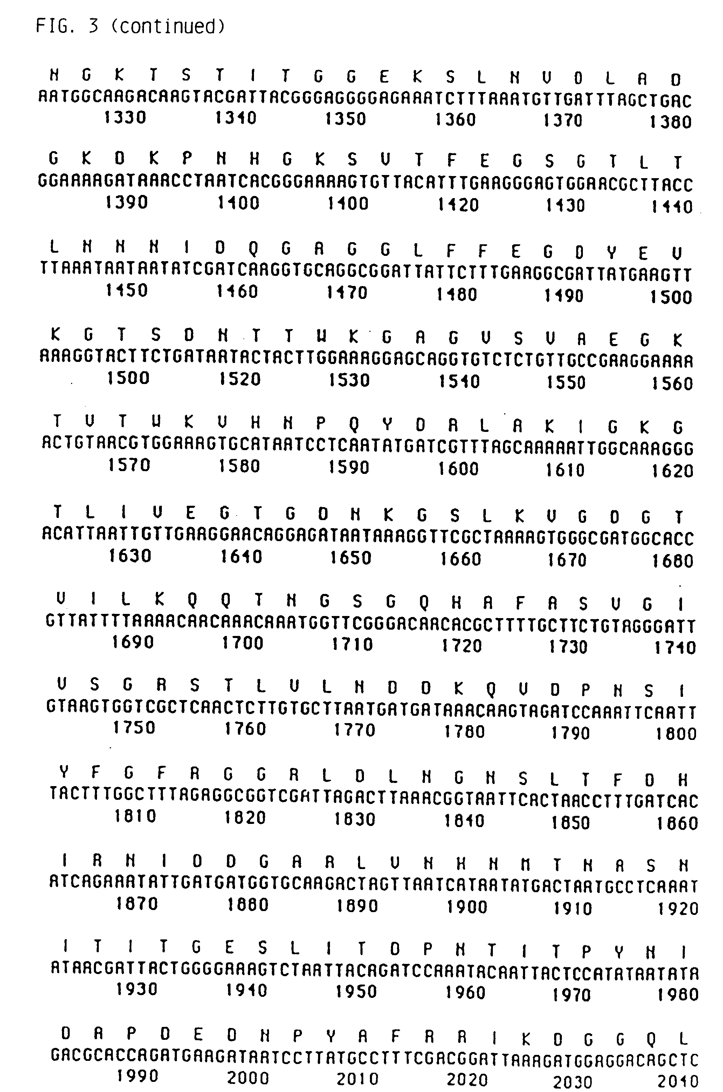

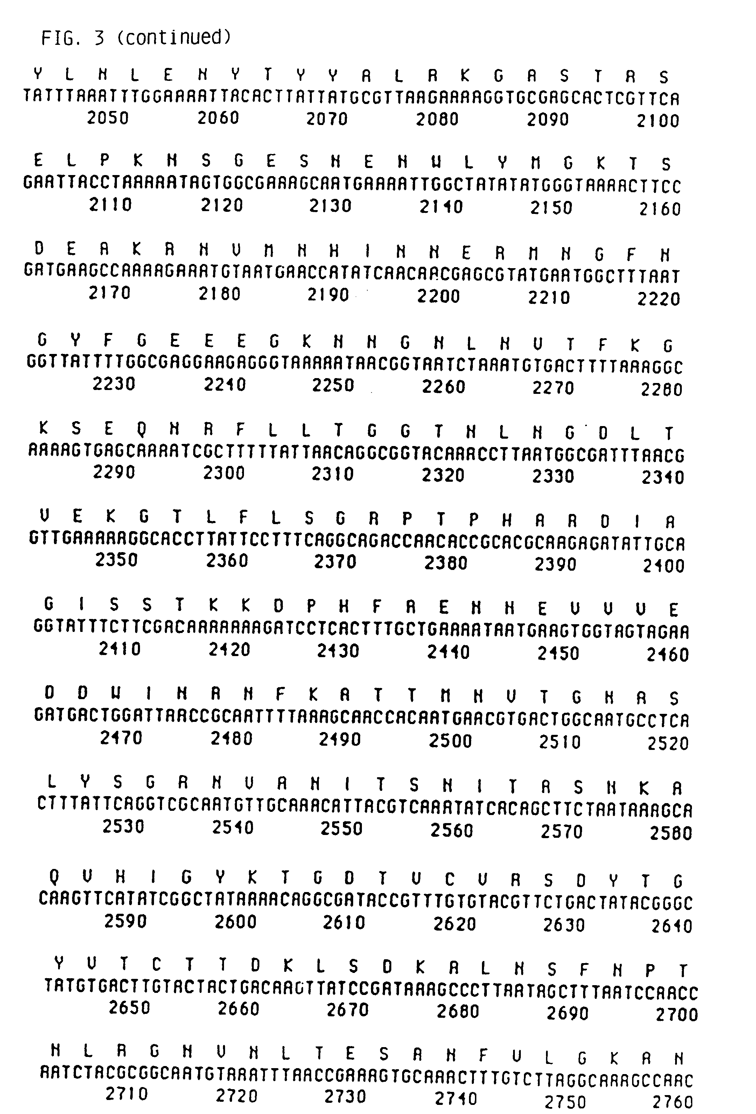

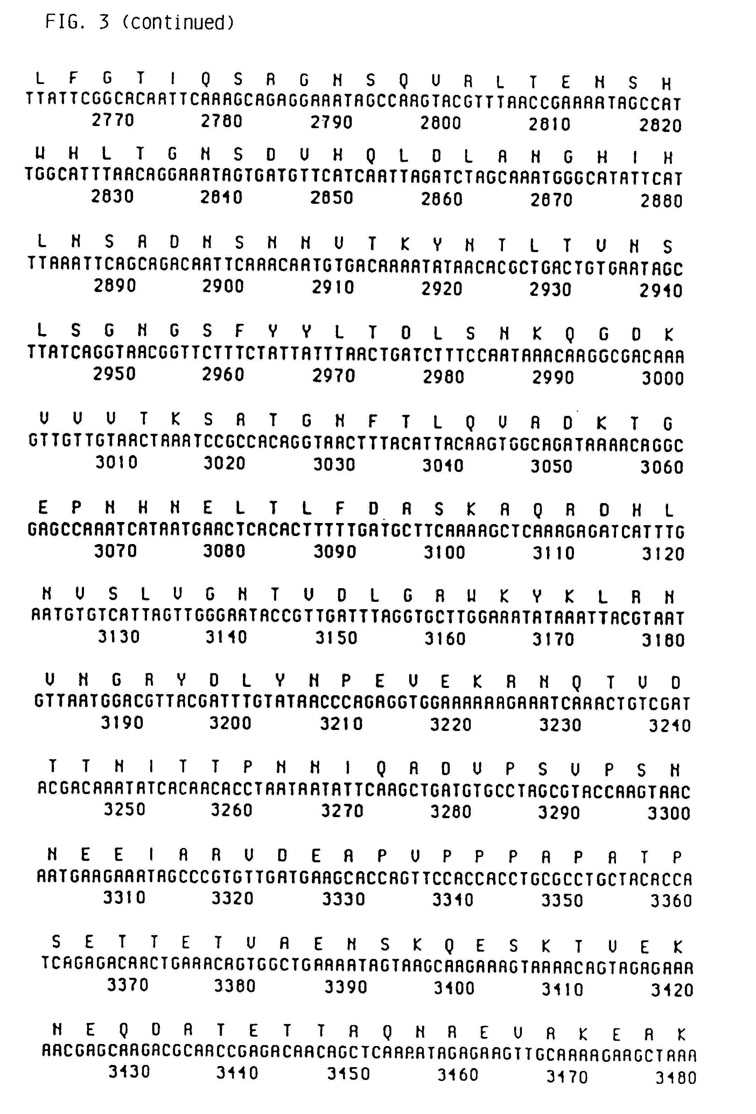

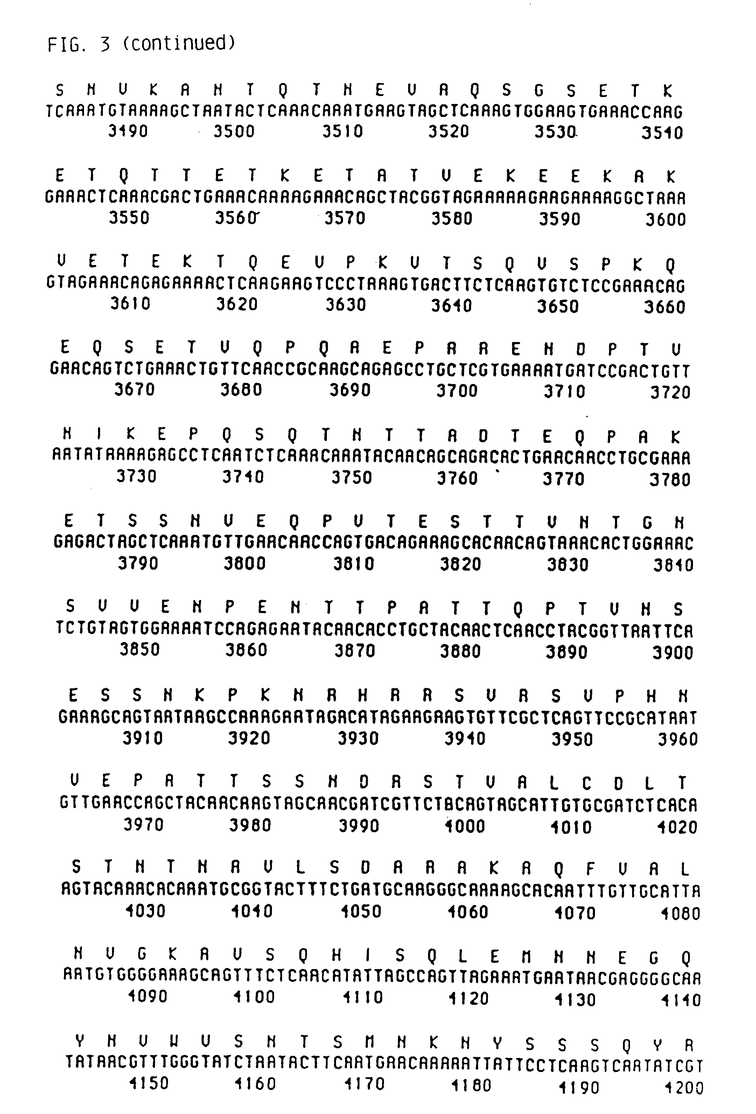

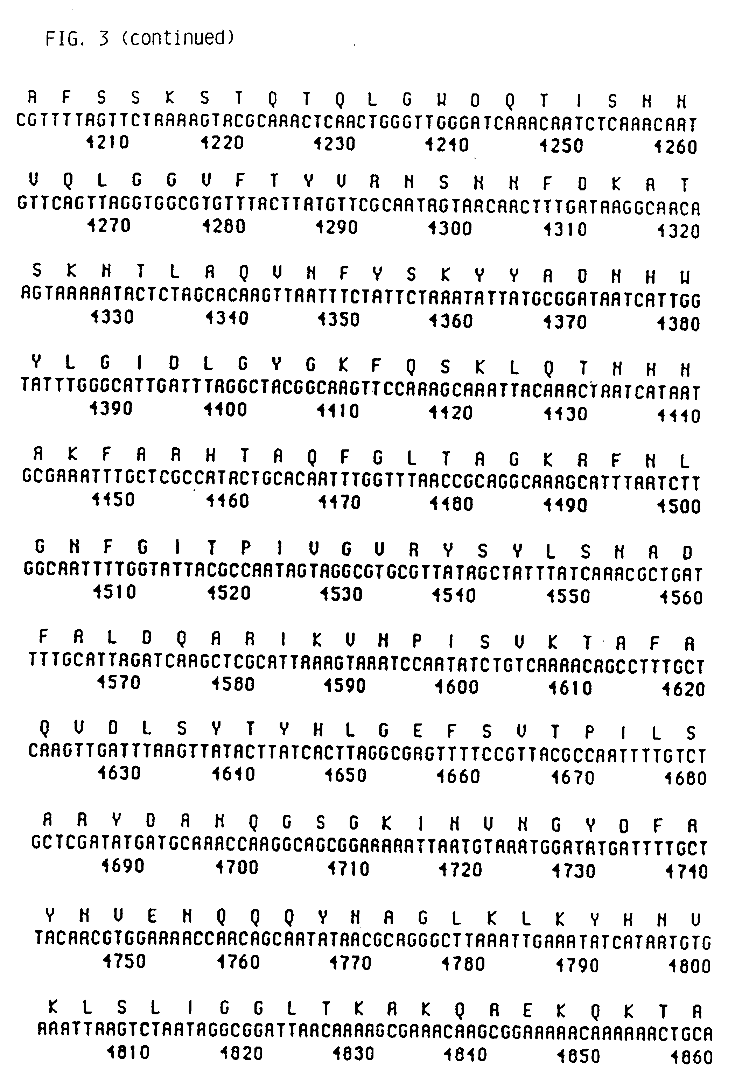

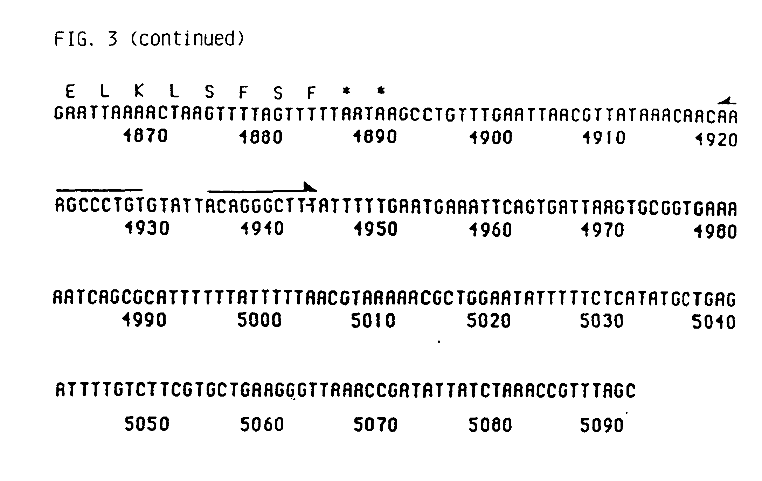

Nucleotide sequence of the iga gene. The 7.5 kb EcoRI/HindIII fragment of λ368iga-16, which contains the iga gene, occasionally was unstable when subcloned into M13mp19 and propagated in E. coli JM109. Therefore, in the sequencing strategy the internal PstI site was used to divide this fragment (see Fig. 2). The sticky ends of the 5.9 kb HindIII/PstI and the 1.6 kb PstI/EcoRI fragments of λ368iga-16 (Fig. 2) were made blunt ended by the Klenow enzyme and subcloned in both orientations into the HincII site of M13mp19. Deletion derivatives of these subclones were generated by Exonuclease III digestion for various times of SacI/BamHI-opened replicative form DNA followed by nuclease S1 treatment and self ligation. The 5091 nucleotide (nt) sequence of the 7.5 kb EcoRI/HindIIII fragment presented in Fig. 3 was obtained by sequencing a total of 79 such deletion clones, which gave overlapping sequences of both strands in this central part of the fragment. The sequence across the internal PstI site, i.e. at nt 4856 to nt 4861 in Fig. 3 was verified by subcloning into M13mp19 and sequencing 300 nucleotides from the 3' end of the 1640 base pair HhaI fragment ranging from nt 3349 to nt 4988.

The sequence revealed a large open reading frame (ORF) with homology to the N. gonorrhoeae iga gene sequence previously published (Pohlner, J. et al, Nature 325, 458-462 (1987)). The suggested iga gene from H. influenzae strain HK368 starting at the first ATG in the ORF as shown in Fig. 3 consists of 4646 nucleotides, including the TAA stop codon, encoding a deduced protein of 1541 amino acids. This primary translation product has a deduced molecular mass of 169 kd in contrast to the estimated size of the mature IgA1 protease of approximately 100 kd. In the discussion below is suggested a scheme for post-translational modifications of the preprotein to account for this discrepancy.

In connection with the present invention the iga gene encoding the IgA1 protease from the H. influenzae serotype b strain HK368 has been cloned and sequenced in order to characterize the protein further and thereby to achieve a better understanding of its role during the bacterial infection. The iga nucleotide sequence presented in Fig. 3 and the deduced primary structure of the IgA1 protease protein reveal several interesting features.

Potential start and stop signals for transcription and translation. The observed expression in E. coli implies that the H. influenzae iga gene must have transcription and translation signals which are recognized by the E. coli cell. A potential ribosome binding Shine-Dalgarno sequence 5'-TAAAGA -3' (nt 248 to nt 253 in Fig. 3) can be found eight nucleotides upstream from the putative translation start at the first ATG in the open reading frame (A at nt 262 in Fig. 3). This sequence has a homology of 5 identical positions out of 6 to the complementary sequence of the 3' end of the E. coli 16 S ribosomal RNA (Shine, J. and Dalgarno, 1, Proc. Natl. Sci. USA 71, 1342-1346 (1974)). In addition, it is located in an acceptable position according to the rules for gene beginnings suggested by Stormo et al., Nucl. Acids Res. 10, 2971-2996 (1982)). The sequence 5'-TAAACT-3' (nt 235 to nt 240) and 5'-TTGTG-3' (nt 211 to nt 215) might constitute the transcription signals at -10 and -35, respectively. The proposed -10 sequence is identical to the E. coli consensus sequence at the four best conserved positions (Hawley, D.K. and Mc Clure, W.R., Nucl. Acids Res. 11, 2237-2255 (1983)).

Located 19 base pairs upstream from this, the -35 sequence is within the allowed spacing and has a homology of 3 out of 5 positions identical to the E. coli consensus sequence (Hawley, D.K. et al., supra). The identification of these three putative promotor elements strongly support the suggestion of translational initiation at nt 262.

The ORF is ended by two sequential TAA stop codons at nt 4885 to nt 4890. A sequence similar to a typical Rho-independent transcription terminator is found at nt 4919 to nt 4943 and contains a perfect inverted repeat with the potential of forming a "hairpin structure", having a loop of 5 nt and a stem of 10 nt including five pairs of CG (Fig. 3). Like the typical E. coli terminators, this sequence structure is followed by a stretch rich in T residues (Rosenberg, M. and Court, D., Ann. Rev. Genet. 13, 314-353 (1979)).

Codon usage. Fig. 3 shows the codon usage of the H. influenzae iga gene. There is a striking tendency for the triplets to end preferentially with A or T. This may be a simple reflection of the high A+T content of the genome (mole percent A+T = 62). The codon frequencies in the iga gene are similar to those observed in four other H. influenzae genes for which the nucleotide sequence is known (Chandrasegaran, s. et al., Gene 70, 387-392 (1988)). A mole percent of 63% A+T was found for the 4626 nt H. influenzae iga gene sequenced, which is close to the published estimate of 62% A+T for the whole genome (Kilian, M., J. Gen. Microbiol. 93, 9-62 (1976)). According to the observations of Bibb et al., Gene 30, 157-166 (1984), genes of this genome should have an A+T content of 51%, 65%, and 69% for the first, second, and third position within codons. Values of 53%, 61%, and 75% A+T were found at these positions.

Based on the preference of T over C in the third base of duet codons and the relatively low quartet to duet frequency ratios among sextet codons encoding Arg, Leu, and Ser, the iga gene should be weakly expressed according to the rules of Grantham et al.,Nucl. Acids Res. 9, 43-74 (1981). This agrees well with the relatively low amount of IgA1 protease protein produced by H. influenzae.

This bias towards A and T residues of the third position of degenerate codons in the H. influenzae iga gene is in contrast to the markedly unbiased codon usage for the iga gene from N. gonorrhoeae which has a genomic A+T content of 51%.

These two genes most likely have a common ancestor indicating that apparently different constraints in codon usage have worked during the evolution of these two organisms.

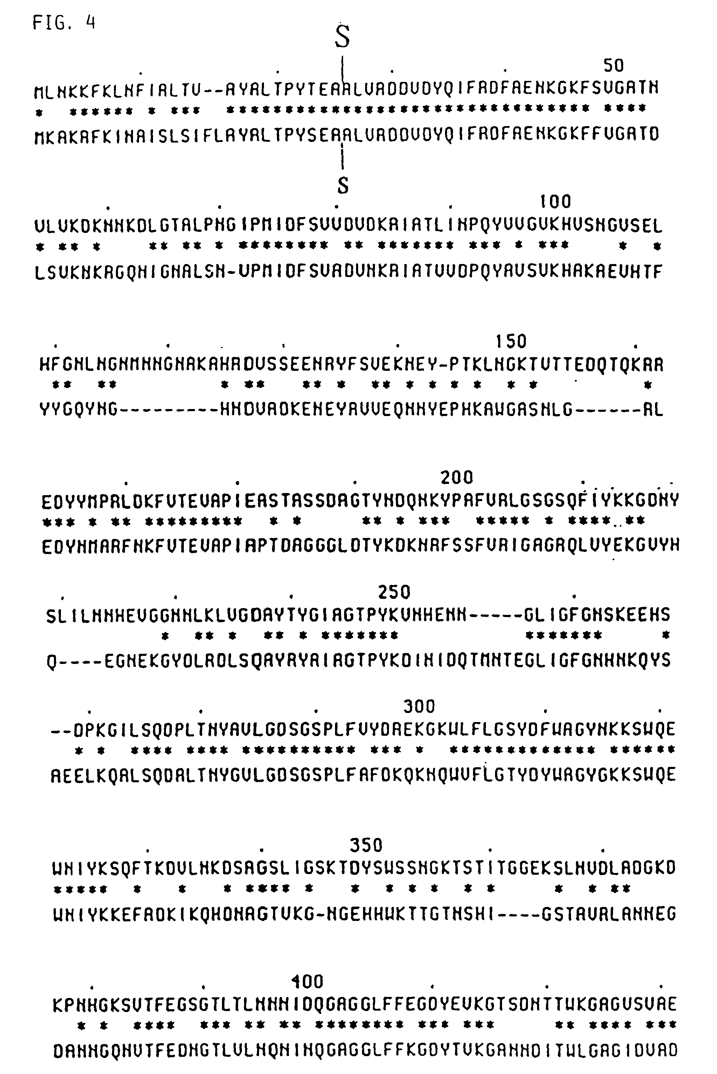

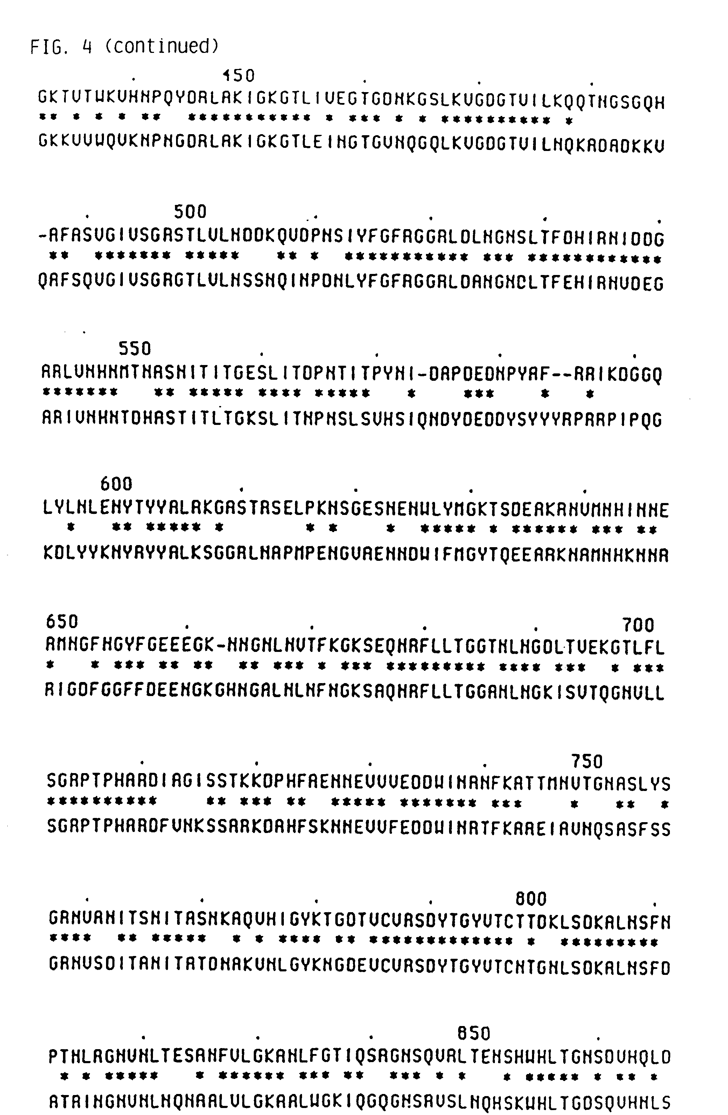

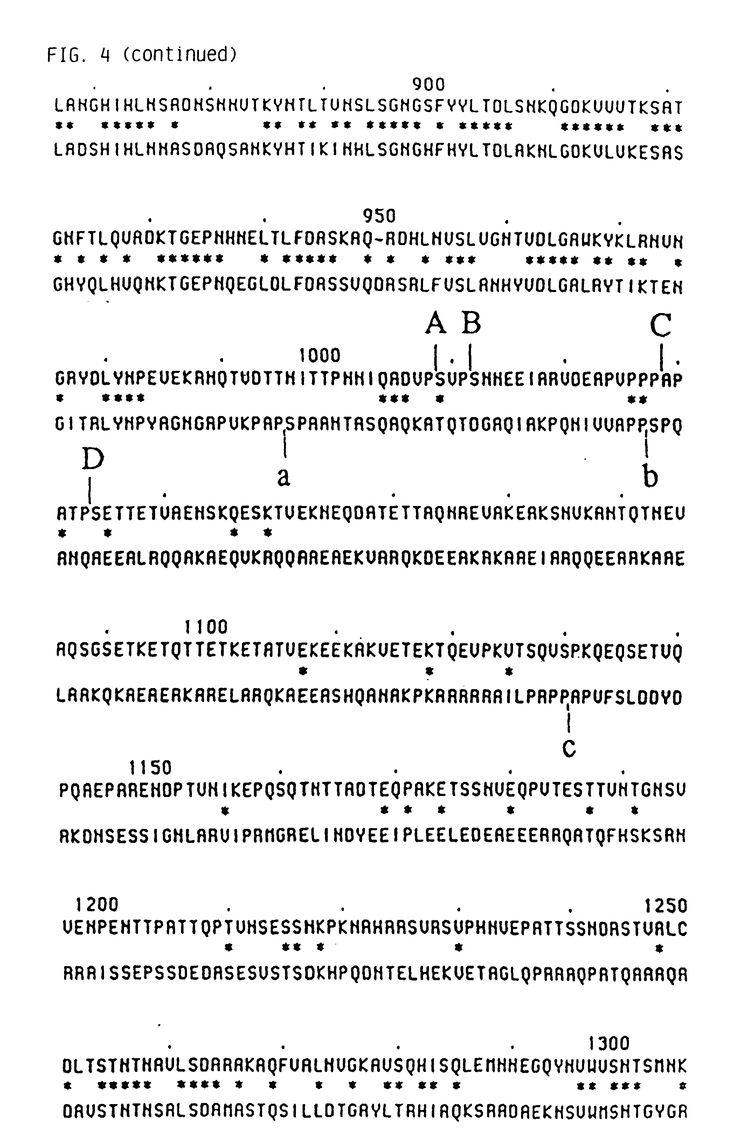

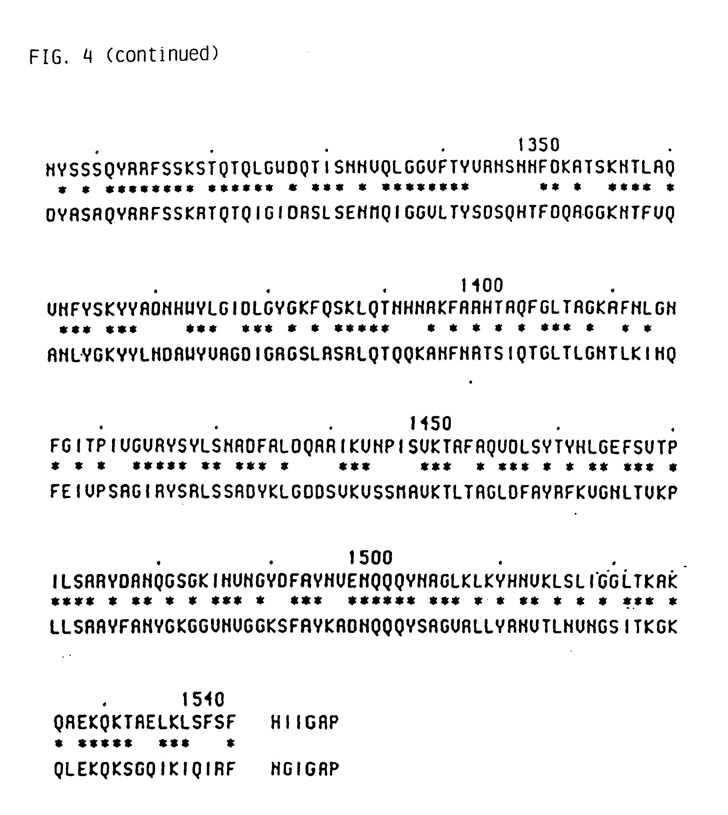

Comparison of H. influenzae and N. gonorrhoeae protease sequences. Koomey and Falkow, Infect. Immun. 43, 101-1-7 (1984) have previously shown that the cloned type 2 N. gonorrhoeae iga gene hybridizes to the H. influenzae type 1 iga gene. The present comparison of the nucleotide sequences of these two iga genes revealed regions with a high degree of homology as well as areas with essentially no homology. The maximal homology between the corresponding deduced amino acid sequences of the two IgA1 protease proteins was obtained by aligning them as shown in Fig. 4.

Pohlner et al., Nature 325, 458-462 (1987) found that the precursor of the N.gonorrhoeae IgA1 protease contains three functional domains; the amino-terminal signal peptide, the central IgA1 protease, and the carboxy-terminal "helper" domain. The leader peptide is released during translocation of the preprotein into the periplasmic space while the helper domain is assumed to create a pore in the outer membrane for excretion of the protease domain and remains associated with the membrane upon autoproteolytic cleavage.

The primary translation product, deduced from the H. influenzae iga sequence presented in Fig. 4, has a molecular mass of 169 kd. The mature type 1 IgA1 protease from H. influenzae has been estimated to be about 100 kd. Grundy et al. (J. Bacteriol. 169, 4442-4450 (1987)) found that a 2.2 to 3.1 kb region of the 3' end of a type 1 IgA1 protease gene from a H. influenzae serotype d strain is necessary for the secretion of the protease but not for its activity. They suggest that this region is cleaved off during maturation of the protease. Based on these observations and the amino acid sequence homologies shown in Fig. 4, it is suggested that the H. influenzae IgA1 protease is secreted by a mechanism similar to the one proposed by Pohlner et al., supra, for the N. gonorrhoeae IgA1 protease.

The aminoterminal part of the H. influenzae IgA1 protease contains positively charged lysines at amino acid (aa) positions 4, 5 and 7 followed by a hydrophobic stretch including the helix breaking proline at position 21 four residues before an alanine (Fig. 4). These features are characteristic of signal sequences which are cleaved off and released with the alanine as the C-terminal amino acid, (Watson, M.E.E., Nucl. Acids Res. 12, 5145-5164 (1984)). The two protease sequences aligned as shown in Fig. 4 are identical around the position of the cleavage site of the N. gonorrhoeae protease leader peptide determined by Pohlner et al., supra. Therefore it is proposed that the 25 amino terminal amino acids of the H. influenzae IgA1 protease preprotein constitute the signal peptide.

Sequences rich in prolines, much like the type 1 IgA1 protease target site in the hinge region of the human IgA1 molecule (Fig. 1), are exclusively found at three positions in the H. influenzae protease sequence (A, B, and D in Fig. 4). A single sequence with homology to the type 2 IgA1 protease target site (Fig. 1) is found in the same region of the protease (C in Fig. 4) It is proposed that one or more of these four sequence elements are functionally equivalent to the autoproteolytic sites a, b, and c in the N. gonorrhoeae protease, as shown in Fig. 4.

When an N-terminal signal sequence of 25 amino acids is taken into account, autoproteolytic cleavage at positions A, B, C or D would result in excreted proteases of deduced molecular masses of 107.8, 108.1, 109.9, or 110.3 kd, respectively. Each of these values are in acceptable agreement with the estimated Mr of 100 kd.

Strikingly, a stretch of 32 amino acids (aa 16 to aa 47) is identical in the two proteases except for a single conservative substitution. This shows that the N-terminal part of the mature IgA1 protease has been evolutionary conserved suggesting that it is essential to the enzymatic function or specificity of the protease molecule.

Another notably well-conserved sequence is found at aa 785 to aa 797. It contains the two conserved cysteines which Pohlner et al.,supra, proposed to be part of the active site in the N.gonorrhoeae protease. A third cysteine is found in the "helper" domain of the H. influenzae protease preprotein at aa position 1250.

In striking contrast to the rest of the H. influenzae IgA1 protease, the region from aa 980 to aa 1240 has no significant sequence homology to the N. gonorrhoeae protease (see Fig. 4). This stretch is proposed to constitute the N-terminal part of the helper domain. It is very hydrophilic in both proteins suggesting that it has a common function in the secretion of the two proteases. No evolutionary deletions or insertions had to be introduced in this divergent area of the two protease sequences to obtain the flanking homologies as shown in Fig. 4. This observation is in contrast to the results of Grundy et al. who found a deletion-substitution loop in this region when analyzing DNA heteroduplexes formed between cloned H. influenzae type 1 and H. influenzae type 2 IgA1 protease genes.

The cloning and sequencing of the IgA1 protease gene from H. influenzae serotype b provides a tool to produce this protein in large quantities for further structural and functional analyses.

As mentioned, the IgA1 protease in question is primarily useful as an active component in vaccines for immunizing against bacterial meningitis. A vaccination against infections of the type concerned is in principle best performed by administering the vaccine via the oral cavity so that the vaccine gets into contact with the lymphoid tissues in the intestinal wall. Examples of administration of the vaccines are a) presentation on the surface of transformed E.coli or other suitable host (vide infra), b) incorporated in micropheres alone or together with other suitable vaccine components or immunostimulant, and c) as part of a fusion protein with the subunit B of the cholera toxin. Administration of the vaccines may also be by the parenteral route. These examples are provided to describe and not to limit the possible applications.

The fact that the signals for expression of IgA1 gene and that the secretion mechanisms of the H. influenzae IgA1 proteases function in E. coli imply that the IgA1 protease gene has potential use as a vector for producing foreign proteins in cultures of E. coli or other suitable hosts.

The following examples are provided to describe this aspect in further detail. The examples are intended to illustrate and not to limit the invention:

Changes in the structural part of the iga gene do not affect secretion of the protein from the surface of the transformed E.coli clone. Insertion of prokaryotic or eukaryotic gene sequences encoding foreign polypeptides into the IgA1 protease gene results in the transport to the E.coli cell surface of a hybrid protein. The hybrid protein may be exposed and retained on the cell surface or released by autoproteolysis into the growth medium depending on the location and size of the foreign gene insert. Such transformed E.coli clones or other suitable hosts exposing or releasing such fusion proteins on or from the cell surface may be used as an oral vaccine for presentation of one or more vaccine polypeptides to relevant lymphoid tissues in the gut of humans or animals. Alternatively, such clones may be employed for production of foreign polypeptides in E.coli or other hosts. Release into the medium may, when necessary, be accomplished by addition of active IgA1 protease.

### Detailed Description of Aspect 2 (Neisseriae meningitidis):

### Bacterial strains

A collection of 133 isolates of Neisseria meningitidis recovered from 97 patients and 36 healthy carriers in 19 countries was examined. The 133 isolates represented 88 multilocus enzyme genotypes (ETs) defined on the basis of electrophoretically detectable allelic variations in 15 genes encoding enzymes (Caugant. D. et al., J. Bacteriol. 169, 2781-2792, 1987; Infect. Immun. 56, 2060-2068, 1988). In addition, 8 gonococcal strains, 4 each of the IgA1 protease types 1 and 2, were examined. IgA1 protease preparations produced by the method described by Higerd, T.B. et al. (J. Immunol. Methods 18, 245-249, 1977) from four meningococcal strains and one gonococcal strain were used to immunize rabbits by a method described previously (Kilian, M. et al. Molec. Immunol. 20, 1051-1058, 1983). The resulting antisera were examined for their ability to inhibit IgA1 proteases from all 133 meningococccal and 8 gonococcal strains using methods described (Kilian, M. et al., supra). While three of the four antisera against meningococcal proteases and the antiserum against gonococcal IgA1 protease inhibited IgA1 proteases from only some of the 141 strains, the remaining antiserum surprisingly caused inhibition of all IgA1 proteases whether of type 1 or 2 and whether of meningococcal or gonococcal origin. The inhibition titer determined against representative IgA1 protease preparations adjusted to standard activity as described (Kilian, M. et al. supra) ranged from 1024 to 4096. The meningococcal strain (HF13) used for production of this antiserum was of serogroup Y, subtype 2c, and produced an IgA1 protease of type 2. Identical IgA1 proteases were identified in other meningoccal strains of subtype 2c.

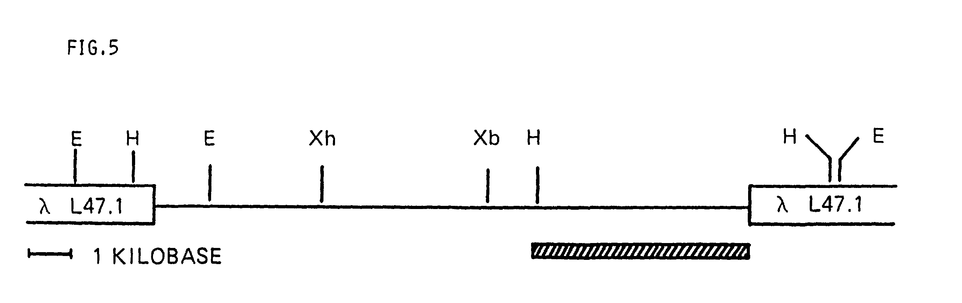

The IgA1 protease gene from Neisseria meningitidis strain HF13 was cloned in E.coli using methods described above for the cloning of the H.influenzae iga gene. A map of the gene and adjoining genome areas in phage lambda L47.1 is shown in fig. 5. The transformed E.coli clone secreted the meningococcal IgA1 protease into the growth medium and the resulting IgA1 protease isolated from the medium was capable of inducing neutralizing antibodies in rabbits with identical inhibition spectrum as antibodies raised against the IgA1 protease of the parental meningococcal strain HF13. These results show that the IgA1 protease isolated from meningococcal strain HF13, from a strain producing an identical or similar IgA1 protease, or from E.coli or other suitable host transformed with the iga gene, is a candidate for a vaccine against all types of meningococci and gonococci.

### Detailed Description of Aspect 3 (Allergic diseases):

The mentioned extensive IgA degradation in nasopharyngeal secretions of children with allergic diseases suggests that IgA1 protease-induced cleavage of IgA is a contributing factor to the development and perpetuation of allergic diseases. Two possible explanations might explain the more extensive IgA cleavage seen in allergic subjects: 1) an increased colonization by IgA1 protease-producing bacteria in the nasopharynx and 2) an inability of allergy-prone subjects to react against bacterial IgA1 proteases with enzyme-neutralizing antibodies.

To study this, the nasopharyngeal flora of 24 children was examined at the age of 1 year and the age of approximately 30 months. Representative bacterial isolates were identified and examined for IgA1 protease activity using standard methods. All children were examined clinically and immunologically to establish a possible diagnosis of allergic diseases.

Out of the 24 children, a total of 11 had developed allergic diseases at the age of 1 year. Table 1 correlates the clinically and immunologically established allergy diagnosis with the bacteriological findings. The figures demonstrate a significantly higher proportion of IgA1 protease-producing bacteria in nasopharyngeal samples from allergic children. Surprisingly, the numerically most significant IgA1 protease-producer was Streptococcus mitis biovar 1. This organism has previously been a member of "Streptococcus mitior" (Kilian, M., Mikkelsen, L., & Henrichsen, J. Int. J. Syst. Bacteriol. 39, 471-484, 1989). This organism occurs in an IgA1 protease-producing form and in a form not producing this enzyme. In accordance with the findings in the control group, previous studies have demonstrated that S.mitis biovar 1 normally occurs in the form lacking IgA1 protease activity in the nasopharynx (Kilian, M. & Holmgren, K. Infect. Immun. 31, 868-873, 1981). At the examination 18 months later, samples from allergic children contained Streptococcus pneumoniae and Streptococcus oralis in addition to bacteria listed in Table 1. These data demonstrate that children developing allergic diseases are colonized with IgA1 protease-producing bacteria in the nasopharynx to a higher extent than healthy children. A vaccine containing IgA1 proteases from the relevant bacteria mentioned is likely to secure that the immune barrier is left intact, thus preventing the development and perpetuation of allergic diseases.

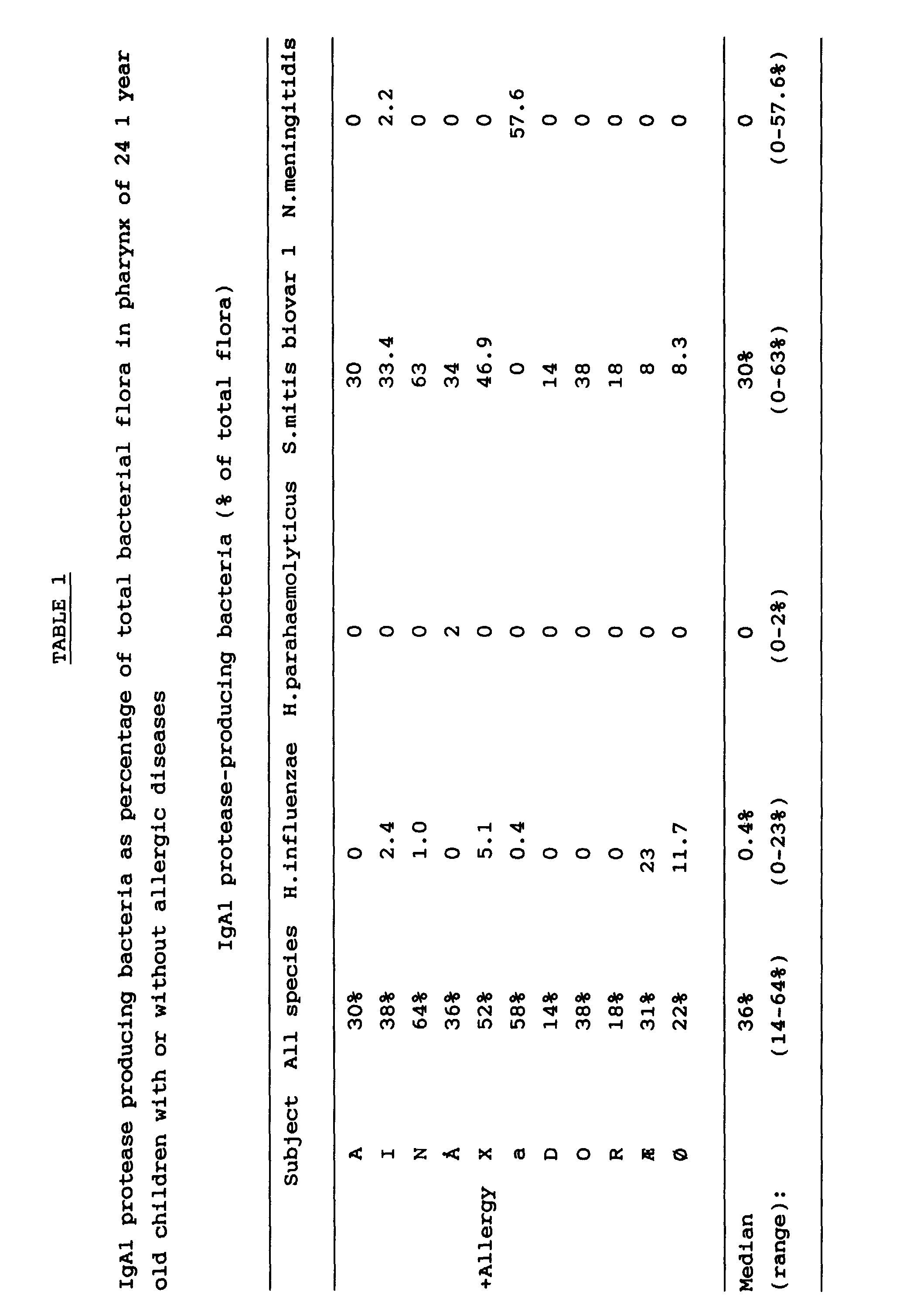

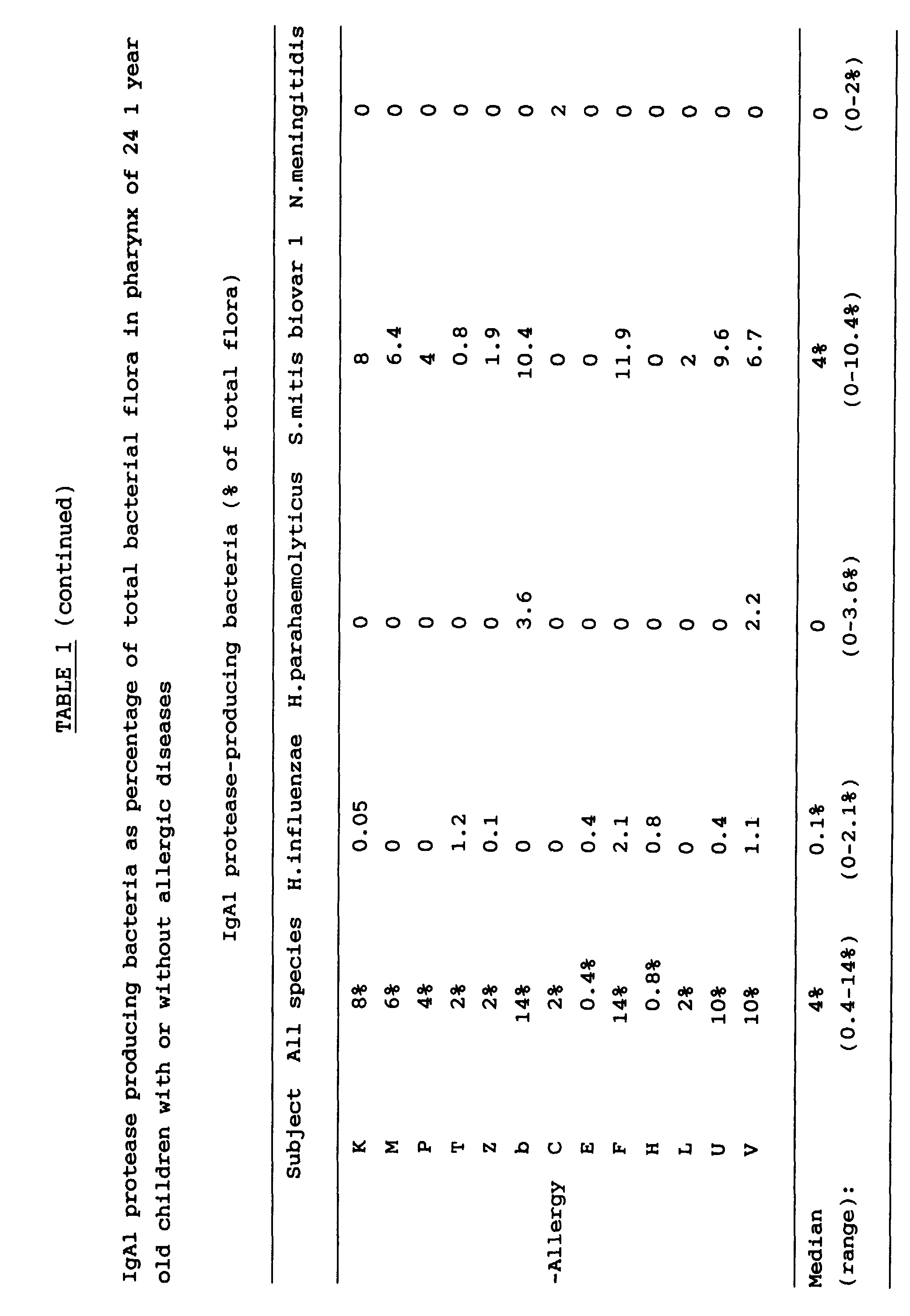

### Explanation to the figures.

Fig. 1. Primary structure of the hinge region of human IgA1. The arrows indicate the peptide bonds cleaved by the individual IgA1 proteases.

Fig. 2. Structure of the H. influenzae serotype b IgA1 protease gene. A Restriction maps of the analysed recombinant lambda phages containing sequences with homology to the H. influenzae serotype d iga gene probe. Symbols: (E) EcoRI; (H) HindIII; terminal bar indicates left arm of the individual phages; (+) or (-) indicate presence or absence of human IgA1 cleaving activity in culture supernatants of E. coli harbouring the individual clones. B Restriction fragments from EcoRI/HindIII double digests hybridizing to the two serotype d iga gene probes. The deduced orientation of the iga gene is indicated by 5' and 3'. C Combined restriction map of λ368iga-8 and λ369iga-16, an extension of A by including the restriction enzymes BamHI (B), ClaI (C), and PstI (P). D Restriction fragments of λ368iga-16 subcloned into M13mp19 for sequence analysis.

Fig. 3. Nucleotide sequence of the cloned iga gene from H. influenzae serotype b strain HK368. The sequence shown is derived from a part of the 7.5 kb EcoRI-HindIII fragment of the λ368iga-16 insert. The deduced amino acid sequence of the large ORF is shown above the nucleotide sequence. The two sequential stop codons are indicated by asterisks. Putative -35 and -10 promotor elements as well as a possible Shine-Dalgarno (SD) sequence are overlined. The possible Rho-independent dual terminator is indicated by divergent arrows.

Fig. 4. Amino acid homologies between the deduced H. influenzae iga gene product (H11GAP) and the N. gonorrhoeae iga gene product (NG1GAP). Sequence data of N. gonorrhoeae iga was derived from Pohlner et al., supra. Asterisks denote identical or functionally equivalent residues. Gaps indicated by dashes are introduced in the two sequences to obtain maximal homology. Cleavage site for the amino terminal signal peptide of the N. gonorrhoeae protease is indicated by s; S denotes the proposed equivalent cleavage site of the H. influenzae protease precursor. Positions a, b, and c represent autoproteolytic sites for the N. gonorrhoeae proteasse; positions A, B, C, and D indicate similar autoproteolytic sites suggested for the H. influenzae protease.

Fig. 5. Physical map of lambda HF13iga-1 expressing the meningococcal IgA1 protease gene from strain HF13. Symbols: E, Eco RI; H, Hind III; Xb, Xba I; Xh, Xho I. Hatched bar, fragment hydridizing to the 4.3 kb Hind III fragment of pVD 105 which contains the major part including the 3' end of the N.gonorrhoeae iga gene (Koomey, J.M., and Falkow, S., 1984 supra). The enzymes BamHi and SalI were found not to cleave the lambda HF13 iga-1 DNA.

## Claims (Claims for the following Contracting State(s): AT, BE, CH, DE, DK, FR, GB, IT, LI, LU, NL, SE)

1. An immunoglobulin A1 protease (IgA1 protease) from Haemophilus influenzae serotype b or immunogenic fragments thereof, obtainable by cloning an IgA1 protease gene, said gene being
an iga gene from Haemophilus influenzae serotype b strain HK 368 having a nucleotide sequence as shown in Fig. 3, and encoding an amino acid sequence as shown in Fig. 3,
and having the ability to induce neutralizing antibodies against IgA1 proteases of pathogenic Haemophilus influenzae serotype b.

2. An IgA1 protease from Neisseria meningitidis or immunogenic fragments thereof, obtainable by cloning an IgA1 protease gene, said gene being
an iga gene from Neisseria meningitidis strain of serogroup Y, subtype 2c (designated HF13), a map of said gene and adjoining genome areas in phage lambda L47 shown in Fig. 5,
and having the ability to induce neutralizing antibodies against IgA1 proteases of a number of known genotypes of pathogenic Neisseria meningitidis.

3. An IgA1 protease from Neisseria meningitidis or immunogenic fragments thereof, obtainable by cloning an IgA1 protease gene, said gene being
an iga gene from Neisseria meningitidis strain or serogroup, subtype 2c (designated HF13), a map of said gene and adjoining genome areas in phage lambda L47 shown in Fig. 5,
and having the ability to induce neutralizing antibodies against IgA1 proteases of a number of known genotypes of pathogenic Neisseria gonorrhoeae.

4. An IgA1 protease from Neisseria meningitidis or immunogenic fragments thereof, obtainable by cloning an IgA1 protease gene, said gene being
an iga gene from Neisseria meningitidis strain or serogroup Y, subtype 2c (designated HF13), a map of said gene and adjoining genome areas in phage lambda L47 shown in Fig. 5,
and having the ability to induce neutralizing antibodies against IgA1 proteases of a number of known genotypes of pathogenic Neisseria meningitidis and Neisseria gonorrhoeae.

5. A method for producing an IgA1 protease according to claim 1, whereby an expression vector containing the iga gene is introduced into an Escherichia coli or other suitable host organism, whereafter said host organism is cultured to produce the IgA1 protease.

6. A gene coding for an IgA1 protease according to claim 1, said gene having the nucleotide sequence shown in Fig. 3.

7. A vaccine for immunizing against bacterial meningitis containing an IgA1 protease according to any of claims 1 to 4.

8. A vaccine according to claim 7, further comprising other suitable vaccine components or immunostimulants.

9. A vaccine according to claim 7 or 8, said vaccine being formulated for administration parenterally or mucosally via the oral cavity.

10. A vaccine for the prevention or treatment of allergic diseases containing IgA1 proteases from bacteria, said proteases being indirectly responsible for the allergic disease as a result of their cleavage of the mucosal antibodies.

11. A vaccine according to claim 10 containing IgA1 proteases from bacteria selected among Haemophilus influenzae, H. parahaemolyticus, Streptococcus pneumoniae, Strepto- H coccus sanguis, Streptococcus oralis, Streptococcus mitis biovar 1 and Neisseria meningitidis.

12. A vaccine according to any of claims 7 to 11, wherein the IgA1 proteases are incorporated in microspheres.

13. A vaccine according to any of claims 7 to 12, in which the IgA protease is present as part of a fusion protein with the subunit B of the cholera toxin.

## Claims (Claims for the following Contracting State(s): ES)

1. A method for producing an immunoglobulin A1 protease (IgA1 protease) or immunogenic fragments thereof, characterized in that an expression vector containing an iga gene coding for the IgA1 protease, said gene originating from Haemophilus influenzae serotype b strain HK 368 having a nucleotide sequence as shown in Fig. 3, and encoding an amino acid sequence as shown in Fig. 3, is introduced into a suitable host organism, preferably selected from Escherichia coli, Salmonella and Saccharomyces, whereafter said host organism is cultured to produce the IgA1 protease, and the protease is isolated from the culture medium.

2. A method for producing an immunoglobulin Al protease (IgA1 protease) or immunogenic fragments thereof, characterized in that an expression vector containing an iga gene coding for the IgA1 protease, said gene originating from Neisseria meningitidis strain of serogroup Y, subtype 2c (designated HF13), a map of said gene and adjoining genome areas in phage lambda L47 shown in Fig. 5, is introduced into Escherichia coli or other suitable host organism, whereafter said host organism is cultured to produce the IgA1 protease, and the protease is isolated from the culture medium.

3. A method according to claim 1 or 2, wherein the host organism is Escherichia coli.

4. A gene coding for an IgA1 protease according to claim 1, said gene having the nucleotide sequence shown in Fig. 3.

## Patentansprüche (Patentansprüche für folgende(n) Vertragsstaat(en): AT, BE, CH, DE, DK, FR, GB, IT, LI, LU, NL, SE)

1. Immunglobulin-A1-Protease (IgA1-Protease) von Hämophilus influenzae Serotyp b oder immunogene Fragmente davon, erhältlich durch Klonieren eines IgA1-Protease-Gens, wobei das Gen
- ein iga-Gen von Hämophilus influenzae Serotyp b, Stamm HK 368 mit der in Figur 3 gezeigten Nukleotidsequenz ist, das eine Aminosäuresequenz, wie in Figur 3 gezeigt, kodiert,
- und die Fähigkeit hat, neutralisierende Antikörper gegen IgA1-Proteasen von pathogenen Hämophilus influenzae Serotyp b zu induzieren.

2. IgA1-Protease von Neisseria meningitidis oder immunogene Fragmente davon, erhältlich durch Klonieren eines IgA1-Protease-Gens, wobei das Gen
- ein iga-Gen eines Neisseria meningitidis-Stamms der Serogruppe Y, Subtyp 2c (bezeichnet mit HF13) ist, wobei eine Karte des Gens und benachbarter Genombereiche in dem Phagen Lambda L47 in Figur 5 gezeigt ist,
- und die Fähigkeit hat, neutralisierende Antikörper gegen IgA1-Proteasen einer Anzahl bekannter Genotypen von pathogenen Neisseria meningitidis zu induzieren.

3. IgA1-Protease von Neisseria meningitidis oder immunogene Fragmente davon, erhältlich durch Klonieren eines IgA1-Protease-Gens, wobei das Gen
- ein iga-Gen eines Neisseria meningitidis-Stamms der Serogruppe Y, Subtyp 2c (bezeichnet mit HF13) ist, wobei eine Karte des Gens und benachbarter Genombereiche in dem Phagen Lambda L47 in Figur 5 gezeigt ist,
- und die Fähigkeit hat, neutralisierende Antikörper gegen IgA1-Proteasen einer Anzahl bekannter Genotypen von pathogenen Neisseria gonorrhoeae zu induzieren.

4. IgA1-Protease von Neisseria meningitidis oder immunogene Fragmente davon, erhältlich durch Klonieren eines IgA1-Protease-Gens, wobei das Gen
- ein iga-Gen eines Neisseria meningitidis-Stamms der Serogruppe Y, Subtyp 2c (bezeichnet mit HF13) ist, wobei eine Karte des Gens und benachbarter Genombereiche in dem Phagen Lambda L47 in Figur 5 gezeigt ist,
- und die Fähigkeit hat, neutralisierende Antikörper gegen IgA1-Proteasen einer Anzahl bekannter Genotypen von pathogenen Neisseria meningitidis und Neisseria gonorrhoeae zu induzieren.

5. Verfahren zur Herstellung einer IgA1-Protease nach Anspruch 1, wobei ein Expressionsvektor, der das iga-Gen enthält, in Escherichia coli oder einen anderen geeigneten Wirtsorganismus eingesetzt wird, wonach der Wirtsorganismus gezüchtet wird, um die IgA1-Protease zu produzieren.

6. Gen, das eine IgA1-Protease nach Anspruch 1 kodiert, wobei das Gen die in Figur 3 gezeigte Nukleotidsequenz hat.

7. Impfstoff zur Immunisierung gegen bakterielle Meningitis enthaltend eine IgA1-Protease nach einem der Ansprüche 1 bis 4.

8. Impfstoff nach Anspruch 7, weiter enthaltend andere geeignete Impfstoff-Komponenten oder Immunstimulantien.

9. Impfstoff nach Anspruch 7 oder 8, wobei der Impfstoff zur parenteralen Verabreichung oder zur Verabreichung über die Schleimhaut in der Mundhöhle formuliert wird.

10. Impfstoff zur Verhütung oder Behandlung allergischer Erkrankungen enthaltend IgA1-Proteasen von Bakterien, wobei die Proteasen indirekt für die allergische Erkrankung verantwortlich sind als Ergebnis der Spaltung der Schleimhaut-Antikörper.

11. Impfstoff nach Anspruch 10, enthaltend IgA1-Proteasen aus Bakterien, ausgewählt aus Hämophilus influenzae, H. parahämolyticus, Streptococcus pneumoniae, Streptococcus sanguis, Streptococcus oralis, Streptococcus mitis biovar 1 und Neisseria meningitidis.

12. Impfstoff nach einem der Ansprüche 7 bis 11, worin die IgA1-Protease in Mikrokügelchen eingearbeitet ist.

13. Impfstoff nach einem der Ansprüche 7 bis 12, worin die IgA1-Protease als Teil eines Fusionsproteins mit der Untereinheit B des Cholera-Toxins vorhanden ist.

## Patentansprüche (Patentansprüche für folgende(n) Vertragsstaat(en): ES)

1. Verfahren zur Herstellung einer Immunoglobulin-A1-Protease (IgA1-Protease) oder immunogener Fragmente davon, dadurch gekennzeichnet, daß ein Expressionsvektor, der ein iga-Gen enthält, das die IgA1-Protease kodiert, wobei das Gen aus Hämophilus influenzae Serotyp b Stamm HK 368 stammt, mit der in Figur 3 gezeigten Nukleotidsequenz, das die in Figur 3 gezeigte Aminosäuresequenz kodiert, in einen geeigneten Wirtsorganismus eingesetzt wird, bevorzugt ausgewählt aus Escherichia coli, Salmonella und Saccharomyces, wonach der Wirtsorganismus gezüchtet wird, um die IgA1-Protease zu erzeugen, und die Protease aus dem Kulturmedium isoliert wird.

2. Verfahren zur Herstellung einer Immunoglobulin-A1-Protease (IgA1-Protease) oder immunogener Fragmente davon, dadurch gekennzeichnet, daß ein Expressionsvektor, der ein iga-Gen enthält, das die IgA1-Protease kodiert, wobei das Gen aus einem Neisseria meningitidis-Stamm der Serogruppe Y, Subtyp 2c (bezeichnet mit HF13) stammt, wobei eine Karte des Gens und benachbarter Genombereiche in dem Phagen Lambda L47 in Figur 5 gezeigt ist, in Escherichia coli oder einen anderen geeigneten Wirtsorganismus eingesetzt wird, wonach der Wirtsorganismus gezüchtet wird, um die IgA1-Protease zu erzeugen, und die Protease aus dem Kulturmedium isoliert wird.

3. Verfahren nach Anspruch 1 oder 2, worin der Wirtsorganismus Escherichia coli ist.

4. Gen kodierend eine IgA1-Protease nach Anspruch 1, wobei das Gen die in Figur 3 gezeigte Nukleotidsequenz hat.

## Revendications (Revendications pour l'(les) Etat(s) contractant(s) suivant(s): AT, BE, CH, DE, DK, FR, GB, IT, LI, LU, NL, SE)

1. Protéase d'immunoglobuline A1 (protéase d'IgA1) d'Haemophilus influenzae sérotype b ou ses fragments immunogènes, que l'on peut obtenir par clonage d'un gène de protéase d'IgA1, ledit gène étant
un gène iga d'Haemophilus influenzae sérotype b souche HK 368 ayant la séquence nucléotidique représentée dans la figure 3, et codant pour la séquence d'acides aminés représentée dans la figure 3,
et ayant l'aptitude à induire des anticorps neutralisants contre les protéases d'IgA1 d'Haemophilus influenzae sérotype b pathogènes.

2. Protéase d'IgA1 de Neisseria meningitidis ou ses fragments immunogènes, que l'on peut obtenir en clonant un gène de protéase d'IgA1, ledit gène étant
un gène iga d'une souche de Neisseria meningitidis de sérogroupe Y, sous-type 2c (appelé HF13), une carte dudit gène et des régions du génome voisines dans le phage lambda L47 étant représentée dans la figure 5,
et ayant l'aptitude à induire des anticorps neutralisants contre les protéases d'IgA1 d'un certain nombre de génotypes connus de Neisseria meningitidis pathogènes.

3. Protéase d'IgA1 de Neisseria meningitidis ou ses fragments immunogènes, que l'on peut obtenir par clonage d'un gène de protéase d'IgA1, ledit gène étant
un gène iga d'une souche de Neisseria meningitidis de sérogroupe Y, sous-type 2c (appelé HF13), une carte dudit gène et des régions du génome voisines dans le phage lambda L47 étant représentée dans la figure 5,
et ayant l'aptitude à induire des anticorps neutralisants contre les protéases d'IgA1 d'un certain nombre de génotypes connus de Neisseria gonorrhoeae pathogènes.

4. Protéase d'IgA1 de Neisseria meningitidis ou ses fragments immunogènes, que l'on peut obtenir en clonant un gène de protéase d'IgA1, ledit gène étant
un gène iga d'une souche de Neisseria meningitidis de sérogroupe Y, sous-type 2c (appelé HF13), une carte dudit gène et des régions du génome voisines dans le phage lambda L47 étant représentée dans la figure 5,
et ayant l'aptitude à induire des anticorps neutralisants contre les protéases d'IgA1 d'un certain nombre de génotypes connus de Neisseria meningitidis et de Neisseria gonorrhoeae pathogènes.

5. Procédé de production d'une protéase d'IgA1 selon la revendication 1, par lequel on introduit un vecteur d'expression contenant le gène iga dans une Escherichia coli ou un autre organisme hôte approprié, après quoi on cultive ledit organisme hôte pour produire la protéase d'IgA1.

6. Gène codant pour une protéase d'IgA1 selon la revendication 1, ledit gène ayant la séquence de nucléotides représentée dans la figure 3.

7. Vaccin pour immuniser contre la méningite bactérienne contenant une protéase d'IgA1 selon l'une quelconque des revendications 1 à 4.

8. Vaccin selon la revendication 7, comprenant en outre d'autres composants de vaccin ou immunostimulants appropriés.

9. Vaccin selon la revendication 7 ou 8, ledit vaccin étant formulé pour l'administration parentérale ou muqueuse via la cavité orale.

10. Vaccin pour la prévention ou le traitement des maladies allergiques contenant des protéases d'IgA1 provenant de bactéries, lesdites protéases étant indirectement à l'origine de la maladie allergique à la suite de leur segmentation des anticorps muqueux.

11. Vaccin selon la revendication 10 contenant des protéases d'IgA1 provenant de bactéries choisies parmi Haemophilus influenzae, H. parahaemolyticus, Streptococcus pneumoniae, Streptococcus sanguis, Streptococcus oralis, Streptococcus mitis biovar 1 et Neisseria meningitidis.

12. Vaccin selon l'une quelconque des revendications 7 à 11, dans lequel les protéases d'IgA1 sont incorporées dans des microsphères.

13. Vaccin selon l'une quelconque des revendications 7 à 12, dans lequel la protéase d'IgA est présente en tant que partie d'une protéine de fusion avec la sous-unité B de la toxine du choléra.

## Revendications (Revendications pour l'(les) Etat(s) contractant(s) suivant(s): ES)

1. Procédé de production d'une protéase d'immunoglobuline A1 (protéase d'IgA1) ou de ses fragments immunogènes, caractérisé en ce qu'on introduit un vecteur d'expression contenant un gène iga codant pour la protéase d'IgA1, ledit gène provenant d'Haemophilus influenzae sérotype b souche H368 ayant la séquence nucléotidique représentée dans la figure 3, et codant pour la séquence d'acides aminés représentée dans la figure 3, dans un organisme hôte approprié, de préférence choisi parmi Escherichia coli, Salmonella et Saccharomyces, après quoi on cultive ledit organisme hôte pour produire la protéase d'IgA1, et on isole la protéase à partir du milieu de culture.

2. Procédé de production d'une protéase d'immunoglobuline A1 (protéase d'IgA1) ou ses fragments immunogènes, caractérisé en ce qu'on introduit un vecteur d'expression contenant un gène iga codant pour la protéase d'IgA1, ledit gène provenant d'une souche de Neisseria meningitidis de sérogroupe Y, sous-type 2c (appelé HF13), une carte dudit gène et des régions du génome adjacentes dans le phage lambda L47 étant représentée dans la figure 5, dans Escherichia coli ou un autre organisme hôte approprié, après quoi on cultive ledit organisme hôte pour produire la protéase d'IgA1, et on isole la protéase du milieu de culture.

3. Procédé selon la revendication 1 ou 2, dans lequel l'organisme hôte est Escherichia coli.

4. Gène codant pour une protéase d'IgA1 selon la revendication 1, ledit gène ayant la séquence nucléotidique représentée dans la figure 3.
